# EUROPEAN PATENT SPECIFICATION

(11) **EP 3 678 598 B1**
(45) Date of publication and mention of the grant of the patent: **19.07.2023**
(21) Application number: 18853863.1
(22) Date of filing: 05.09.2018
(51) Int. Cl.: A61F 2/24

(54) **INTEGRAL FLUSHING SOLUTION FOR BLOOD STASIS PREVENTION IN ARTIFICIAL HEART VALVES**
INTEGRALE SPÜLLÖSUNG ZUR VERMEIDUNG VON BLUTSTAUUNGEN BEI KÜNSTLICHEN HERZKLAPPEN
SOLUTION DE RINÇAGE COMPLET DESTINÉE À PRÉVENIR LA STASE SANGUINE DANS DES VALVULES CARDIAQUES ARTIFICIELLES

(30) Priority: 07.09.2017 US 201762555548 P; 31.08.2018 US 201816119317
(43) Date of publication of application: 15.07.2020
(73) Proprietor: Edwards Lifesciences Corporation, Irvine, CA 92614 (US)
(72) Inventor: SAAR, Tomer, Irvine, CA 92614 (US); COHEN-TZEMACH, Hanoch, Irvine, CA 92614 (US); BEN ZAKEN, Nadav, Irvine, CA 92614 (US)
(74) Representative: Eisenführ Speiser
(86) International application number: PCT/US2018/049479
(87) International publication number: WO 2019/050900

(56) References cited:
- EP-B1- 0 338 179
- WO-A1-2006/004679
- US-A- 4 872 875
- US-A1- 2004 225 352
- US-A1- 2017 014 229
- US-B1- 6 200 340

## Description

### RELATED APPLICATIONS

This application claims priority to and the benefit of U.S. Provisional Application No. 62/555,548, filed on September 7, 2017, entitled "Integral Flushing Solution for Blood Stasis Prevention in Artificial Heart Valves".

### FIELD OF THE INVENTION

The present invention relates to prosthetic valves, more specifically to a prosthetic valve, such as a surgically implanted valve or a transcatheter heart valve (THV), including a secondary valve and/or flushing valve.

### BACKGROUND OF THE INVENTION

Implantable prosthetic valves can be used to treat various valvular disorders. For example, native heart valves (the aortic, pulmonary, tricuspid and mitral valves) function to prevent backward flow or regurgitation, while allowing forward flow. These heart valves can be rendered less effective by congenital, inflammatory, infectious conditions, etc. Such conditions can eventually lead to serious cardiovascular compromise or death. Doctors have attempted to treat such disorders with surgical repair or replacement of the valve using open heart surgery or percutaneous and minimally invasive surgical approaches.

Transcatheter heart valves can be percutaneously introduced in a compressed state on a catheter and expanded to the desired position by balloon inflation, mechanical expansion, or by utilization of a self-expanding frame or stent. In some cases, transcatheter heart valves, such as surgically implanted valves or THVs might be subjected to blood stasis behind the artificial heart valve leaflets. US 2017/014229 A1 discloses a prosthetic valve comprising a frame and a leaflet structure.

### SUMMARY OF THE DISCLOSURE

This summary is meant to provide examples and is not intended to limit the scope of the invention in any way. For example, any feature included in an example of this summary is not required by the claims, unless the claims explicitly recite the feature. The description discloses exemplary embodiments of prosthetic valves, such as surgically implantable prosthetic valves, and trans-catheter implantable valves. The prosthetic valves can be constructed in a variety of ways.

As defined in independent claim 1, a prosthetic valve has a frame, a primary valve and at least one secondary valve. The primary valve is formed by a leaflet structure. The primary valve is mounted inside the frame. The at least one secondary valve is connected to at least one leaflet of the primary valve. The at least one secondary valve has a stationary portion and a detached portion. At least one aperture is provided in the at least one leaflet and proximate to the secondary valve. In certain exemplary embodiments, the stationary portion is connected to at least one leaflet. In certain exemplary embodiments, the stationary portion is connected to an inner portion of the at least one leaflet and a detached portion contacts a perimeter portion of the at least one leaflet.

Further according to the prosthetic valve as defined in claim 1, when the leaflet structure closes, the secondary valve closes and covers the aperture. When the leaflet structure opens, the secondary valve opens creating one or more secondary flow paths through the aperture(s). In certain exemplary embodiments, the prosthetic valve further includes a skirt positioned between the leaflet structure and the frame. In certain exemplary embodiments, the prosthetic valve further includes a reinforcing strip, wherein the leaflet structure is between the reinforcing strip and the skirt.

In certain exemplary embodiments, the skirt contacts a lower portion of the internal side of the frame. In certain exemplary embodiments, the skirt contacts at least a partial portion of the internal side of the frame. In certain exemplary embodiments, the skirt contacts 3 to 5 mm of the internal side of the frame. In certain exemplary embodiments, the skirt contacts the external wall of the frame.

As defined in independent claim 6, a prosthetic valve includes a frame and a primary valve that includes a disconnected portion. A leaflet structure forms the primary valve and is preferably mounted inside the frame at an attachment line. The disconnected portion of at least one leaflet of the leaflet structure is preferably located at the attachment line near an outer perimeter. As defined in claim 6, when the leaflet structure closes to impede fluid flow through the primary valve, the disconnected portion of the leaflet(s) closes also. As also defined in claim 6, when the leaflet structure opens to allow fluid flow through the primary valve, the disconnected portion of the leaflet(s) opens also.

In certain exemplary embodiments, the prosthetic valve further includes a skirt positioned between the leaflet structure and the frame. In certain exemplary embodiments, the disconnected portion is not attached to the frame.

Various features as described elsewhere in this disclosure can be included in the examples summarized here and various methods and steps for using the examples and features can be used, including as described elsewhere herein.

Further understanding of the nature and advantages of the disclosed inventions can be obtained from the following description and claims, particularly when considered in conjunction with the accompanying drawings in which like parts bear like reference numerals.

### BRIEF DESCRIPTION OF THE DRAWINGS

Further understanding of the nature and advantages of the disclosed inventions can be obtained from the following description and claims, particularly when considered in conjunction with the accompanying drawings in which like parts bear like reference numerals.

To further clarify various aspects of embodiments of the present disclosure, a more particular description of the certain embodiments will be made by reference to various aspects of the appended drawings. It is appreciated that these drawings depict only typical embodiments of the present disclosure and are therefore not to be considered limiting of the scope of the disclosure. Moreover, while the figures may be drawn to scale for some embodiments, the figures are not necessarily drawn to scale for all embodiments. Embodiments of the present disclosure will be described and explained with additional specificity and detail through the use of the accompanying drawings.
FIG. 1 is a side view of an exemplary embodiment of a prosthetic heart valve;
FIG. 2 is a perspective view of the prosthetic valve of FIG. 1 in a closed condition;
FIG. 3 is a perspective view of the prosthetic valve of FIG. 1 in an open condition;
FIG. 4 is a top plan view of another example of a prosthetic valve in a closed condition;
FIGS. 5A to 5D are sectional views of a prosthetic valve;
FIG. 6 is a top plan view of a prosthetic valve;
FIG. 7 is a sectional view of a prosthetic valve;
FIG. 8 is a top plan view of an exemplary prosthetic valve in a diastole phase;
FIG. 9 is a sectional view taken along the plane indicated by line A-A of the exemplary prosthetic valve of FIG. 8;
FIG. 10 is a top plan view of an exemplary prosthetic valve in a systole phase;
FIG. 11 is a sectional view taken along the plane indicated by line B-B of the exemplary prosthetic valve of FIG. 10;
FIG. 12 is a top plan view of an exemplary prosthetic valve in a diastole phase;
FIG. 13 is a sectional view taken along the plane indicated by line C-C of the exemplary prosthetic valve of FIG. 12;
FIG. 14 is a top plan view of an exemplary prosthetic valve in a systole phase;
FIG. 15 is a sectional view taken along the plane indicated by line D-D of the exemplary prosthetic valve of FIG. 14;
FIG. 16 is a top plan view of an exemplary prosthetic valve in a diastole phase;
FIG. 17 is a sectional view taken along the plane indicated by line E-E of the exemplary prosthetic valve of FIG. 16;
FIG. 18 is a top plan view of an exemplary prosthetic valve in a systole phase; and
FIG. 19 is a sectional view taken along the plane indicated by line F-F of the exemplary prosthetic valve of FIG. 18.

### DETAILED DESCRIPTION

The following description refers to the accompanying drawings, which illustrate specific embodiments of the invention. Other embodiments having different structures and operation do not depart from the scope of the present invention. Exemplary embodiments of the present disclosure are directed to prosthetic valves, such as surgically implanted valves and transcatheter heart valves (THVs), that include a secondary valve or flushing valve.

Various examples of THVs and frames are disclosed herein, and any combination of these options may be made unless specifically excluded. For example, any of the secondary valves or flushing valves disclosed, can be used with any type of implantable device, valve, and/or delivery system, even if a specific combination is not explicitly described. In short, individual components of the disclosed systems can be combined with other systems and/or components unless mutually exclusive or otherwise physically impossible.

Transcatheter heart valves or surgically implanted valves might be subjected to blood stasis behind the artificial heart valve leaflets. To prevent blood from pooling and/or remaining stagnant, anti-coagulants are generally used. The present disclosure describes secondary or flushing valves used in a prosthetic valve, including without limitation a transcatheter heart valve. As disclosed herein, a secondary valve or a flushing valve can be used to prevent blood stasis behind the artificial heart valve leaflets. Thus, the use of prescription anti-coagulants can be avoided or reduced.

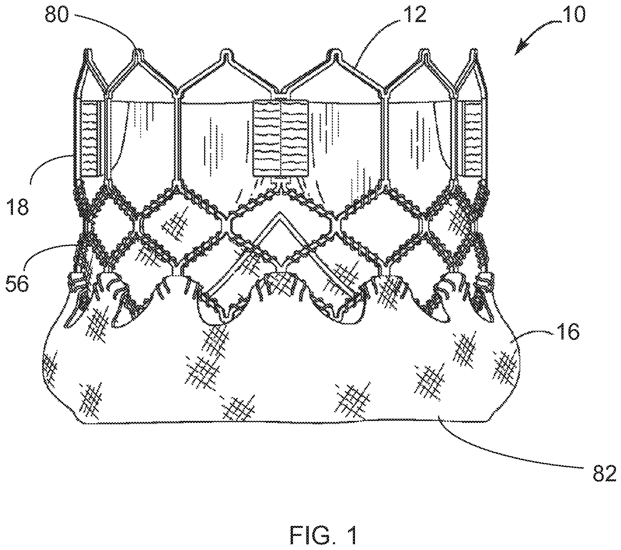

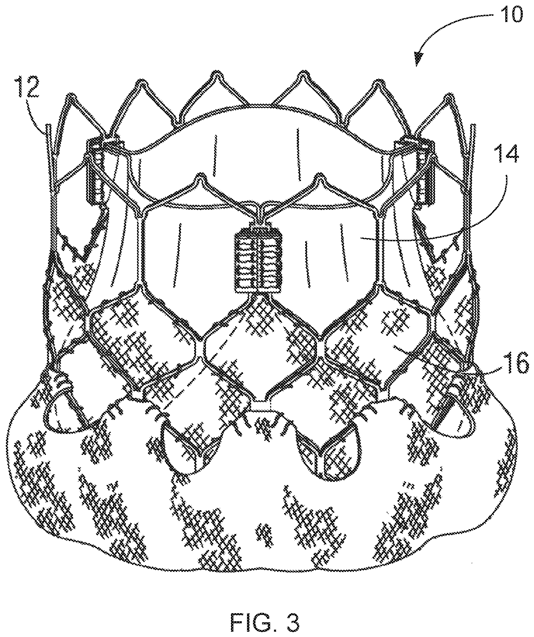

FIGS. 1-3 illustrate a prosthetic valve 10 that can include a secondary or flushing valve. However, a wide variety of different valves can include a secondary or flushing valve. For example, U.S. Pat. Nos. 9,393,110; 7,993,394; 5,411,522; and 6,730,118 disclose non-limitations examples of collapsible transcatheter heart valves that can include a secondary or flushing valve. FIGS. 1-3 are taken from U.S. Patent No. 9,393,110. The primary valve can include the leaflet structure 14 and the frame. In the example illustrated by FIGS 1-3, valve 10 in the illustrated embodiment generally comprises a frame or stent 12, a primary valve defined by a leaflet structure 14 that is supported by the frame, and an optional skirt 16 secured to the frame or stent 12. Valve 10 typically is implanted in the annulus of the native aortic valve but also can be adapted to be implanted in other native valves of the heart or in various other ducts or orifices of the body. Valve 10 has a "lower" end 80 and an "upper" end 82.

Valve 10 and frame 12 are optionally configured to be radially collapsible to a collapsed or crimped state for introduction into the body on a delivery catheter and radially expandable to an expanded state for implanting the valve at a desired location in the body (e.g., the native aortic valve). Frame 12 can be made of an expandable material that permits crimping of the valve to a smaller profile for delivery and expansion of the valve using an expansion device such as the balloon of a balloon catheter. Exemplary expandable materials that can be used to form the frame are described below. In certain exemplary embodiments, valve 10 and/or frame 12 can be mechanically expandable, having a small profile for delivery that can be expanded mechanically using a wide variety of mechanisms. Alternatively, valve 10 can be a self-expanding valve wherein the frame is made of a self-expanding material such as Nitinol. A self-expanding valve can be crimped to a smaller profile and held in the crimped state with a restraining device such as a sheath covering the valve. When the valve is positioned at or near the target site, the restraining device is removed to allow the valve to self-expand to its expanded, functional size.

Suitable expandable materials that can be used to form the frame include, without limitation, stainless steel, a nickel based alloy (e.g., a nickel-cobalt-chromium alloy), polymers, or combinations thereof. In particular embodiments, frame 20 is made of a nickel-cobalt-chromium-molybdenum alloy, such as MP35N^{™} (tradename of SPS Technologies), which is equivalent to UNS R30035 (covered by ASTM F562-02). MP35N^{™}/UNS R30035 comprises 35% nickel, 35% cobalt, 20% chromium, and 10% molybdenum, by weight.

Referring again to FIG. 1, the optional skirt 16 can be formed, for example, of polyethylene terephthalate (PET). The skirt 16 can be secured to the frame 12 via sutures 56, as shown in FIG. 1. Leaflet structure 14 can be attached to the skirt via a thin PET reinforcing strip (or sleeve), which enables a secure suturing and protects the pericardial tissue of the leaflet structure from tears. Leaflet structure 14 can be formed of bovine pericardial tissue, biocompatible synthetic materials, or various other suitable natural or synthetic materials as known in the art and described in U.S. Pat. No. 6,730,118.

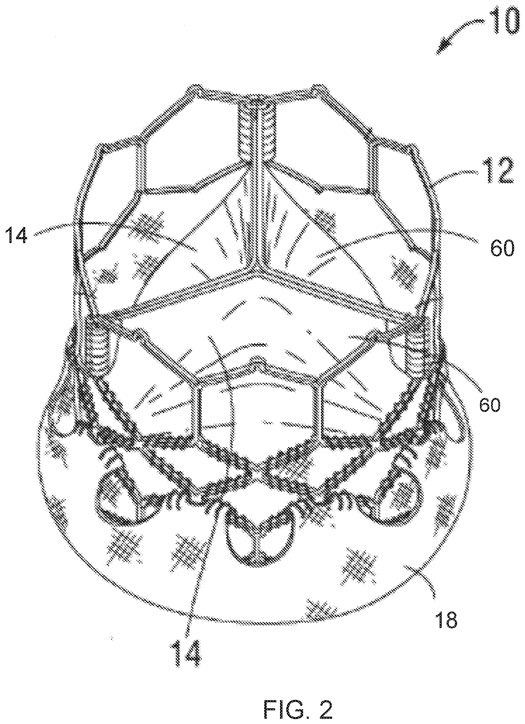

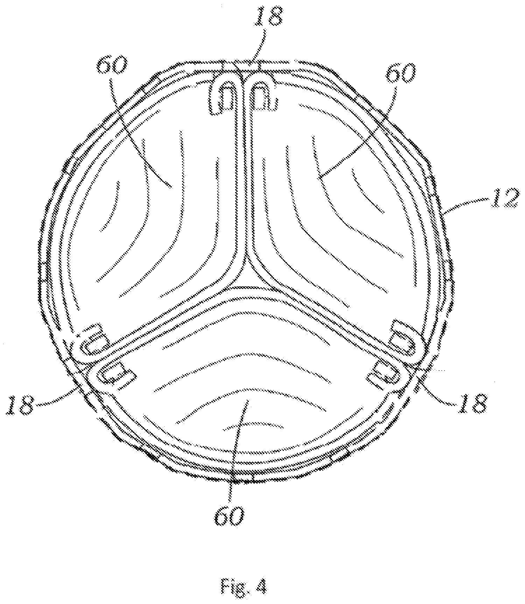

Leaflet structure 14 can comprise three leaflets 60, which can be arranged to collapse in a tricuspid arrangement, as best shown in FIGS. 2 and 4. The prosthetic valve 10 can take a wide variety of different forms. For example, FIG. 4 is taken from US Patent No. 7,993,394 and shows another version of a THV. FIG. 4 shows a top view of the valve assembly attached to frame 12. Leaflets 60 are shown in a generally closed position. As shown, the commissures of the leaflets are aligned with and secured to vertical struts 18 of the frame.

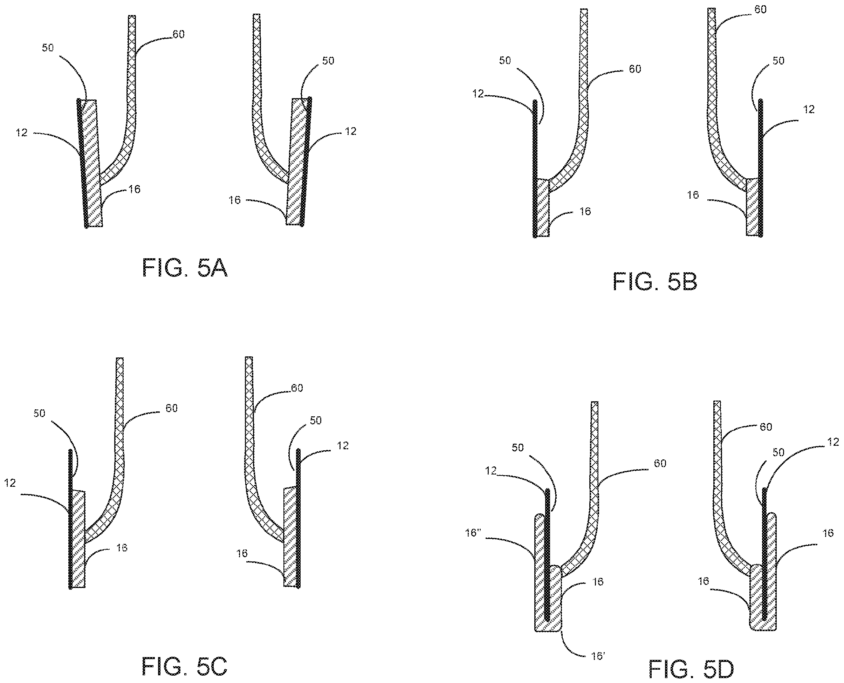

FIGS. 5A to 5D are cross sectional views that illustrate examples of skirts for the valve 10, in addition to the skirt example illustrated by FIGS. 1-3. Each skirt 16 prevents passage of blood through spaces of the frame and/or between the frame and the native tissue where the valve is implanted. The skirt 16 can be connected to the frame 12 in a wide variety of ways. In an exemplary embodiment illustrated by FIG. 5A, the skirt 16 contacts the entire internal side 50 of the frame 12. In another exemplary embodiment illustrated by FIG. 5B, the skirt 16 only contacts the lower part of the frame 12. In the exemplary embodiment illustrated by FIG. 5C, the skirt 16 partially contacts the internal side of the frame 12. In certain exemplary embodiments, the skirt 16 can also be used to fasten the valve 10 inside the frame. As illustrated in FIG. 5D, the skirt 16 is extended at its lower end 16' into an external cover 16" which contacts the external wall of the frame 12.

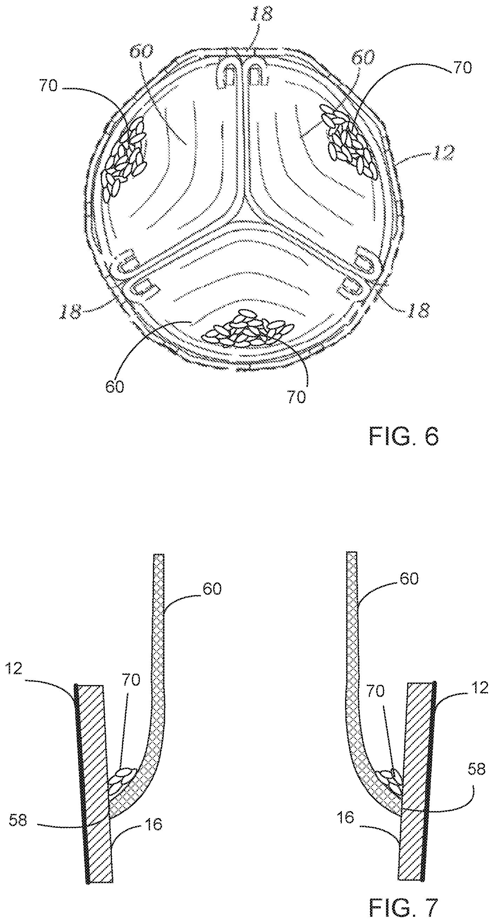

As illustrated in FIGS. 6 and 7, blood stasis can occur behind an exemplary prosthetic valve having leaflets 60. When this occurs, blood 70 can pool and/or remain stagnant behind the leaflets 60. Incorporating a secondary valve and/or flushing valve, into the leaflet at or near the bottom of the leaflet edge allows blood flow during systole phase to flush the bottom end of the "flow pocket" behind the leaflet. In certain exemplary embodiments, the secondary flushing valves shown herein have a flap structure. During the diastole phase, back pressure on the valve closes the secondary valves by pressing the flaps against the leaflets. During the systole phase, forward flow through the valve opens both the primary and secondary valves, creating flow paths near the perimeter of the valve to flush out potentially stagnant pools of blood that may collect behind the leaflets.

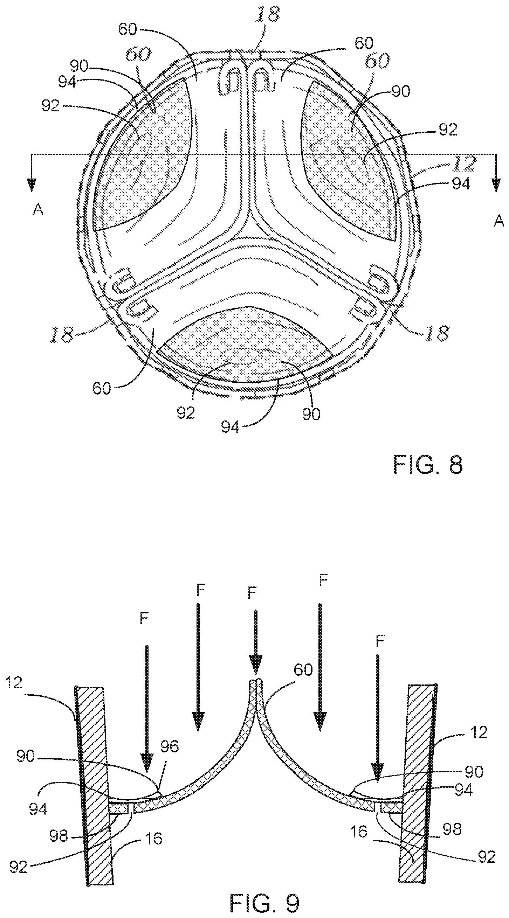

In an embodiment of the present invention, a secondary valve element 90 and an aperture 92 are provided on at least one of the leaflets 60 to allow a small amount of fluid flow therethrough. (See FIGS. 8 and 9.) As illustrated in FIGS. 8 and 9, during the diastole phase, when the leaflets 60 close to impede the flow of fluid in direction F through the valve, the secondary valve element 90 also closes against the leaflet 60 and prevents fluid flow through the aperture 92. FIG. 9 is a sectional view taken along the plane indicated by line A-A of FIG. 8. In certain exemplary embodiments, a stationary portion 94 of the secondary valve element 90 is attached to the skirt 16. In another exemplary embodiment, the stationary portion 94 of the secondary valve element 90 is attached to the outer perimeter 98 of the leaflet 60. During the diastole phase, a detached portion 96 of the secondary valve 90 rests freely against the leaflet 60 forming a seal over the aperture 92, preventing fluid flow through the aperture 92.

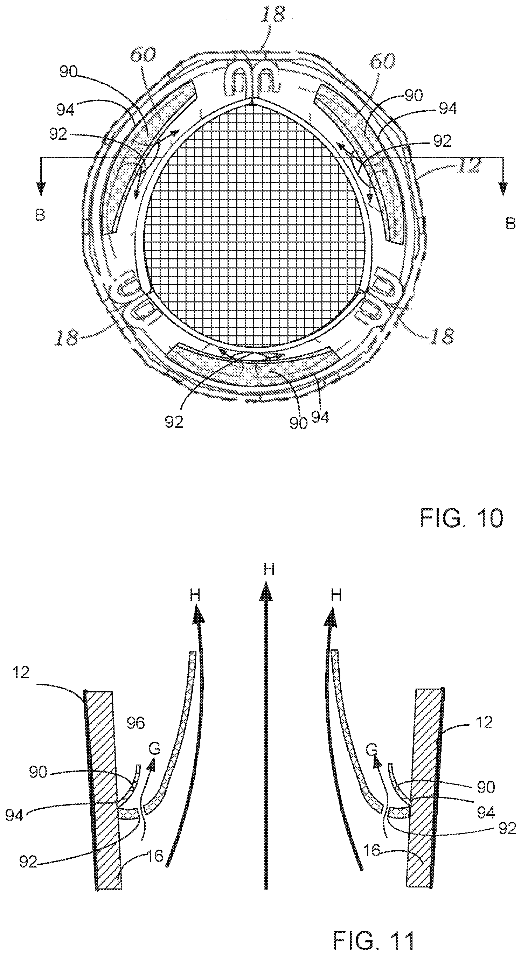

FIGS. 10 and 11 illustrate the valve of Figures 8 and 9 during the systole phase. FIG. 11 is a sectional view taken along the plane indicated by line B-B of the exemplary prosthetic valve of FIG. 10. During the systole phase, forward flow of fluid opens both the leaflets 60 of the primary valve and the secondary valve element 90. Primary flow paths H open the leaflets 60 of the primary valve. Opening the secondary valve element 90 creates secondary flow paths G through the apertures 92. The detached portion 96 of the secondary valve element 90 separates from the leaflet 60 forming a gap over the aperture 92, allowing fluid flow through the aperture 92. The detached portion 96 of the secondary valve element 90 retracts in the direction of the skirt 16 and/or frame 12 and away from the leaflet 60. Thus, the secondary flow paths G flush out potentially stagnant pools of fluid that may have collected behind the leaflet. Thus, blood pooling and/or stagnation can be avoided or greatly reduced.

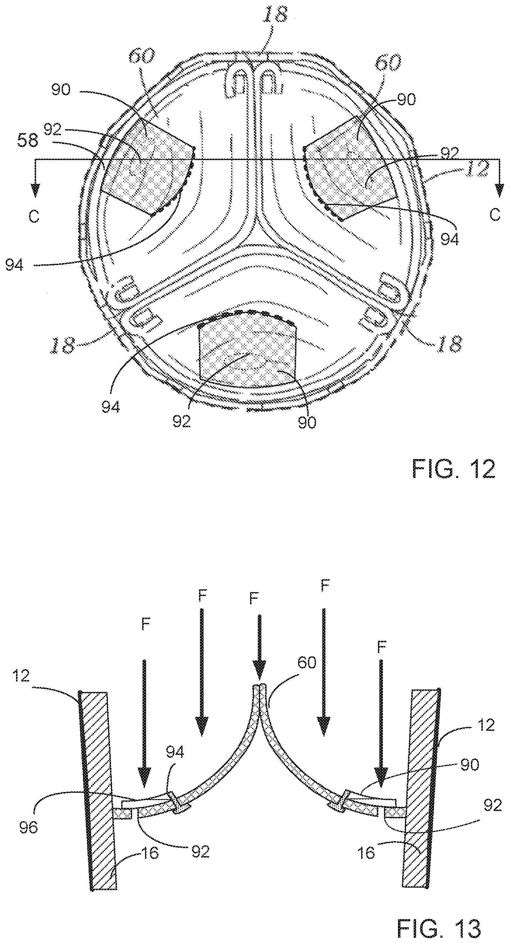

In another exemplary embodiment of the present invention, the secondary valve element 90 is connected to an inner portion of the leaflet 60. FIGS. 12 and 13 illustrate an embodiment having the secondary valve element 90 connected to an inner portion of the leaflet 60. Specifically, FIG. 13 illustrates a sectional view taken along the plane indicated by line C-C of the exemplary prosthetic valve of FIG. 12. During the diastole phase, when the leaflets 60 close to impede the flow of fluid in direction F through the valve, the secondary valve element 90 also closes against the leaflet and prevents fluid flow. The secondary valve element covers the aperture 92 during the diastole phase, preventing fluid flow through the aperture 92. In the exemplary embodiment illustrated in FIGS. 12-15, the stationary portion 94 of the secondary valve 90 is attached to an inner portion of the leaflet 60 and a detached portion 96 of the secondary valve 90 rests freely on the leaflet 60. In certain exemplary embodiments, the detached portion contacts or is close to an outer perimeter of the leaflet. The detached portion is not attached to the frame or the skirt. During the diastole phase, a detached portion 96 of the secondary valve element 90 is pressed against the leaflet 60 by the blood, forming a seal over the aperture 92 and preventing fluid flow through the aperture 92.

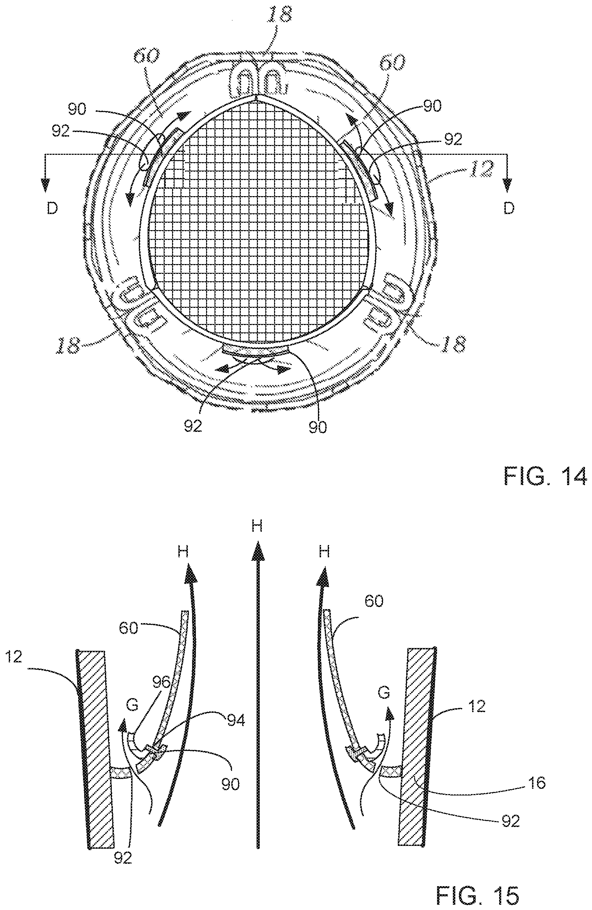

FIGS. 14 and 15 illustrate the valve of FIGS 12 and 13 in the systole phase. FIG. 15 is a sectional view taken along the plane indicated by line D-D of the exemplary prosthetic valve of FIG. 14. During the systole phase, forward flow of fluid opens both the leaflets 60 of the primary valve and the secondary valve element 90. In FIGS. 14 and 15, the primary valve can include leaflets 60. The primary valve can also include the frame. Primary flow paths H open the leaflets 60 of the primary valve. Opening the secondary valve elements 90 creates secondary flow paths G through the apertures 92. The fluid flow moves the secondary valve elements 90 in the direction of the leaflet 60 and away from the skirt 16 and/or frame 12 to an open position. The detached portion 96 of the secondary valve 90 separates from the leaflet 60 forming a gap over the aperture 92, allowing fluid flow through the aperture 92. Thus, the secondary flow paths G flush out potentially stagnant pools of fluid that may have collected behind the leaflet. Thus, blood pooling and/or stagnation can be avoided or greatly reduced.

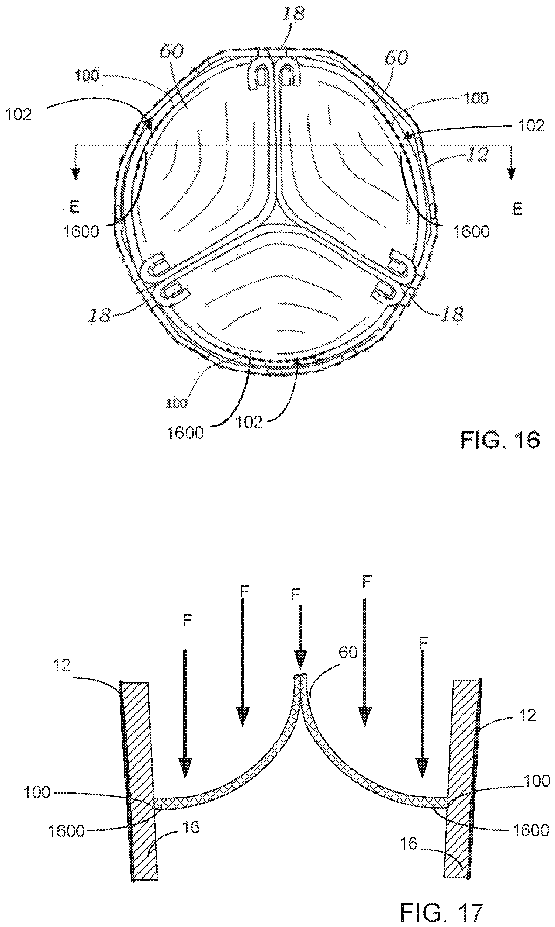

Referring to FIGS. 16 and 17, in another embodiment, a flushing valve 102 comprises an opening or slit 100 that traces a portion of the perimeter of at least one leaflet 60. In the exemplary embodiment, the slit 100 is provided in the leaflet 60 at the outer perimeter by not attaching a portion of the outer perimeter of the leaflet to the skirt 16 or the frame 12. FIG. 17 illustrates a sectional view taken along the plane indicated by line E-E of the exemplary prosthetic valve of FIG. 16. During the diastole phase, when the leaflets 60 close to impede the flow of fluid in direction F through the valve, the disconnected portion 1600 that forms the slit 100 of the leaflet 60 contacts the skirt 16 and thus prevents fluid flow.

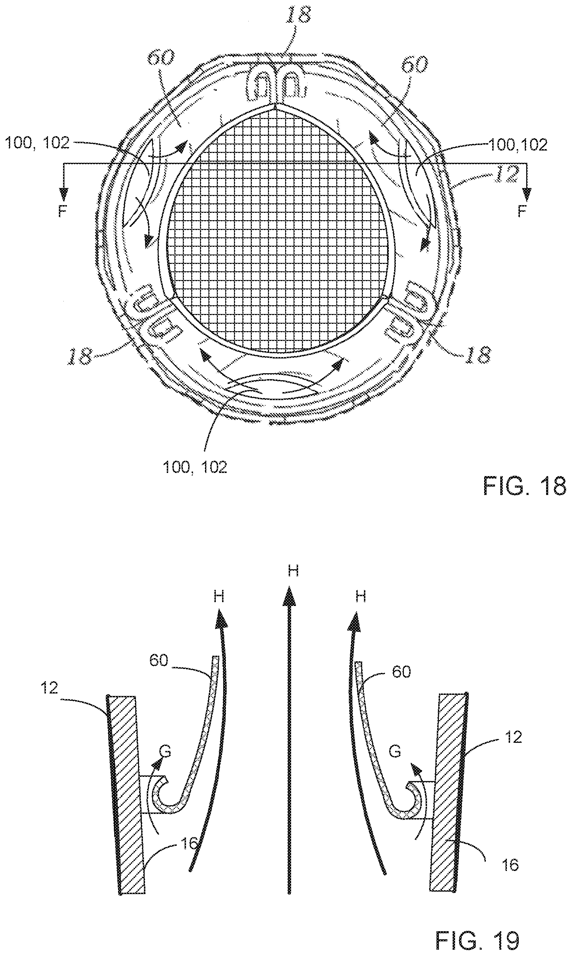

FIGS. 18 and 19 illustrate the valve of FIGS. 16 and 17 in the systole phase. FIG. 19 is a sectional view taken along the plane indicated by line F-F of the exemplary prosthetic valve of FIG. 18. During the systole phase, forward flow of fluid opens both the leaflets 60 of the primary valve and the disconnected portion 1600 that forms the slit 100 of the leaflet 60. Primary flow paths H open the leaflets 60 at the primary valve. A secondary flow path G is created by flow through the flushing valve 102 formed by the slit 100 of the leaflet 60. The disconnected portion that forms the slit 100 of the leaflet 60, retracts in the direction toward the center of the valve 60. The secondary flow paths G flush out potentially stagnant pools of fluid that may have collected behind the leaflet. Thus, blood pooling and/or stagnation can be avoided or greatly reduced.

The secondary flow path G can be in various positions on the leaflet 60. In certain exemplary embodiments, the secondary flow path G is near the perimeter of the leaflet 60. The slit 100 and the flushing valve 102 formed therefrom can be in various positions on the leaflet 60. In certain exemplary embodiments, the slit 100 and the flushing valve 102 formed therefrom are near the perimeter of the leaflet 60.

In view of the many possible embodiments to which the principles of the disclosed invention may be applied, it should be recognized that the illustrated embodiments are only preferred examples of the invention and should not be taken as limiting the scope of the invention. The scope of the invention is defined by the following claims. We therefore claim as our invention all that comes within the scope of these claims.

## Claims

1. A prosthetic valve (10) comprising:
a frame (12);
a leaflet structure (14) having at least one leaflet (60) forming a primary valve mounted inside the frame (12);
at least one secondary valve element (90) connected to at least one leaflet (60) of the leaflet structure (14), wherein the at least one secondary valve element (90) has a stationary portion (94) and a detached portion (96); and
at least one aperture (92) in the at least one leaflet (60) and proximate to the secondary valve element (90);
wherein when the leaflet structure (14) closes, the secondary valve element (90) closes and covers the aperture (92);
wherein when the leaflet structure (14) opens, the secondary valve element (90) moves to create a secondary flow path (G) through the aperture (92).

2. The prosthetic valve of claim 1, wherein the stationary portion (94) is connected to a perimeter (98) of the leaflet structure (14).

3. The prosthetic valve of claim 1, wherein the stationary portion (94) is connected to an inner portion of the at least one leaflet (60).

4. The prosthetic valve of any of claims 1 to 3, further comprising a skirt (16) positioned between the leaflet structure (14) and the frame (12).

5. The prosthetic valve of claim 4, wherein the skirt (16) contacts at least a portion of an internal side (50) of the frame (12).

6. A prosthetic valve (10) comprising:
a frame (12);
a leaflet structure (14) having leaflets (60) forming a primary valve mounted inside the frame (12);
wherein a disconnected portion (1600) of at least one leaflet (60) of the leaflet structure (14) is not attached to the frame (12);
wherein when the leaflet structure (14) closes to impede fluid flow through the primary valve, the disconnected portion (1600) of the leaflet (60) closes;
wherein when the leaflet structure (14) opens to allow fluid flow through the primary valve, the disconnected portion (1600) of the leaflet (60) opens.

7. The prosthetic valve of claim 6, wherein the disconnected portion (1600) is located near an outer perimeter (98) of the leaflet structure (14).

8. The prosthetic valve of any of claims 6 to 7, further comprising a skirt (16) positioned between the leaflet structure (14) and the frame (12).

9. The prosthetic valve of claim 8, wherein the disconnected portion (1600) forms a slit (100) that traces a portion of an outer perimeter (96) of the at least one leaflet (60).

10. The prosthetic valve of claim 9, when dependent on claim 8, wherein when the leaflets (60) close to impede fluid flow through the primary valve, the disconnected portion (1600) contacts the skirt (16) and thus prevents fluid flow.

11. The prosthetic valve of any of claims 6 to 10, wherein the leaflet structure (14) is mounted inside the frame (12) at an attachment line.

12. The prosthetic valve of claim 11, wherein the disconnected portion (1600) of the at least one leaflet (60) of the leaflet structure (14) is located at the attachment line near an outer perimeter (96).

13. The prosthetic valve of any of claims 6 to 12, wherein the valve and the frame (12) are configured to be radially collapsible to a collapsed or crimped state for introduction into the body on a delivery catheter and radially expandable to an expanded state for implanting the valve at a desired location in the body.

14. The prosthetic valve of any of claims 6 to 13, wherein the leaflet structure (14) is formed of bovine pericardial tissue or biocompatible synthetic materials.

## Patentansprüche

1. Klappenprothese (10), umfassend:
einen Rahmen (12);
eine Klappensegelstruktur (14), die wenigstens ein Klappensegel (60) aufweist, die eine Primärklappe bildet, welche im Rahmen (12) montiert ist;
mindestens ein sekundäres Klappenelement (90), das mit mindestens einem Klappensegel (60) der Klappensegelstruktur (14) verbunden ist, wobei das mindestens eine sekundäre Klappenelement (90) einen feststehenden Abschnitt (94) und einen losgelösten Abschnitt (96) aufweist; und
mindestens eine Öffnung (92) in dem mindestens einen Klappensegel (60) und nahe des sekundären Klappenelements (90);
wobei, wenn die Klappensegelstruktur (14) schließt, das sekundäre Klappenelement (90) schließt und die Öffnung (92) abdeckt;
wobei, wenn die Klappensegelstruktur (14) öffnet, sich das sekundäre Klappenelement (90) bewegt, um einen sekundären Strömungspfad (G) durch die Öffnung (92) zu erzeugen.

2. Klappenprothese nach Anspruch 1, wobei der feststehende Abschnitt (94) mit einem Umfang (98) der Struktur des Klappensegels (14) verbunden ist.

3. Klappenprothese nach Anspruch 1, wobei der feststehende Abschnitt (94) mit einem inneren Abschnitt des mindestens einen Klappensegels (60) verbunden ist.

4. Klappenprothese nach einem der Ansprüche 1 bis 3, weiter umfassend eine Schürze (16), die zwischen der Klappensegelstruktur (14) und dem Rahmen (12) positioniert ist.

5. Klappenprothese nach Anspruch 4, wobei die Schürze (16) mindestens einen Abschnitt einer inneren Seite (50) des Rahmens (12) berührt.

6. Klappenprothese (10), umfassend:
einen Rahmen (12);
eine Klappensegelstruktur (14), die Klappensegel (60) aufweist, die eine Primärklappe bildet, welche im Inneren des Rahmens (12) montiert ist;
wobei ein unverbundener Abschnitt (1600) zumindest eines Klappensegels (60) der Klappensegelstruktur (14) nicht an dem Rahmen (12) angebracht ist;
wobei, wenn die Klappensegelstruktur (14) schließt, um eine Fluidströmung durch die Primärklappe zu verhindern, der getrennte Abschnitt (1600) des Klappensegels schließt;
wobei, wenn die Klappensegelstruktur (14) öffnet, um eine Fluidströmung durch die Primärklappe zu ermöglichen, der getrennte Abschnitt (1600) des Klappensegels (60) öffnet.

7. Klappenprothese nach Anspruch 6, wobei der unverbundene Abschnitt (1600) nahe eines äußeren Umfangs (98) der Klappensegelstruktur (14) gelegen ist.

8. Klappenprothese nach einem der Ansprüche 6 bis 7, weiter umfassend eine Schürze (16), die zwischen der Klappensegelstruktur (14) und dem Rahmen (12) positioniert ist.

9. Klappenprothese nach Anspruch 8, wobei der unverbundene Abschnitt (1600) einen Schlitz (100) bildet, der entlang eines Abschnitts eines äußeren Umfangs (96) des mindestens einen Klappensegels (60) verläuft.

10. Klappenprothese nach Anspruch 9, wenn abhängig von Anspruch 8, wobei, wenn die Klappensegel (60) schließen, um Fluidströmung durch die Primärklappe zu verhindern, der unverbundene Abschnitt (1600) die Schürze (16) berührt und somit eine Fluidströmung verhindert.

11. Klappenprothese nach einem der Ansprüche 6 bis 10, wobei die Klappensegelstruktur (14) innerhalb des Rahmens (12) an einer Anbringungslinie montiert ist.

12. Klappenprothese nach Anspruch 11, wobei der unverbundene Abschnitt (1600) des mindestens einen Klappensegels (60) der Klappensegelstruktur (14) an der Anbringungslinie nahe eines äußeren Umfangs (96) gelegen ist.

13. Klappenprothese nach einem der Ansprüche 6 bis 12, wobei die Klappe und der Rahmen (12) dafür ausgebildet sind, radial in einen kollabierten oder gecrimpten Zustand kollabierbar zu sein, zur Einführung in den Körper auf einem Zuführkatheter und radial in einen expandierten Zustand expandierbar sind, zur Implantation der Klappe an einer bevorzugten Stelle in dem Körper.

14. Klappenprothese nach einem der Ansprüche 6 bis 13, wobei die Klappensegelstruktur (14) aus Rinderperikardgewebe oder biokompatiblen synthetischen Materialien gebildet ist.

## Revendications

1. Valvule prothétique (10) comprenant :
un cadre (12) ;
une structure de feuillet (14) présentant au moins un feuillet (60) formant une valvule primaire montée à l'intérieur du cadre (12) ;
au moins un élément de valvule secondaire (90) relié à au moins un feuillet (60) de la structure de feuillet (14), dans lequel l'au moins un élément de valvule secondaire (90) présente une partie fixe (94) et une partie détachée (96) ; et
au moins une ouverture (92) dans l'au moins un feuillet (60) et à proximité de l'élément de valvule secondaire (90) ;
dans laquelle, lorsque la structure de feuillet (14) se ferme, l'élément de valvule secondaire (90) se ferme et recouvre l'ouverture (92) ;
dans laquelle, lorsque la structure de feuillet (14) s'ouvre, l'élément de valvule secondaire (90) se déplace pour créer une voie d'écoulement secondaire (G) à travers l'ouverture (92).

2. Valvule prothétique selon la revendication 1, dans laquelle la partie fixe (94) est reliée à un périmètre (98) de la structure de feuillet (14).

3. Valvule prothétique selon la revendication 1, dans laquelle la partie fixe (94) est reliée à une partie intérieure de l'au moins un feuillet (60).

4. Valvule prothétique selon l'une quelconque des revendications 1 à 3, comprenant en outre une collerette (16) positionnée entre la structure de feuillet (14) et le cadre (12).

5. Valvule prothétique selon la revendication 4, dans laquelle la collerette (16) est en contact avec au moins une partie d'un côté intérieur (50) du cadre (12).

6. Valvule prothétique (10) comprenant :
un cadre (12) ;
une structure de feuillet (14) présentant des feuillets (60) formant une valvule primaire montée à l'intérieur du cadre (12) ;
dans laquelle une partie déconnectée (1600) d'au moins un feuillet (60) de la structure de feuillet (14) n'est pas attachée au cadre (12) ;
dans laquelle, lorsque la structure de feuillet (14) se ferme pour empêcher l'écoulement du fluide à travers la valvule primaire, la partie déconnectée (1600) du feuillet (60) se ferme ;
dans laquelle, lorsque la structure de feuillet (14) s'ouvre pour permettre l'écoulement du fluide à travers la valvule primaire, la partie déconnectée (1600) du feuillet (60) s'ouvre.

7. Valvule prothétique selon la revendication 6, dans laquelle la partie déconnectée (1600) est située près d'un périmètre extérieur (98) de la structure de feuillet (14).

8. Valvule prothétique selon l'une quelconque des revendications 6 à 7, comprenant en outre une collerette (16) positionnée entre la structure de feuillet (14) et le cadre (12).

9. Valvule prothétique selon la revendication 8, dans laquelle la partie déconnectée (1600) forme une fente (100) qui trace une partie d'un périmètre extérieur (96) de l'au moins un feuillet (60).

10. Valvule prothétique selon la revendication 9, lorsqu'elle dépend de la revendication 8, dans laquelle, lorsque les feuillets (60) se ferment pour empêcher l'écoulement du fluide à travers la valvule primaire, la partie déconnectée (1600) entre en contact avec la collerette (16) et empêche ainsi l'écoulement du fluide.

11. Valvule prothétique selon l'une quelconque des revendications 6 à 10, dans laquelle la structure de feuillet (14) est montée à l'intérieur du cadre (12) au niveau d'une ligne de fixation.

12. Valvule prothétique selon la revendication 11, dans laquelle la partie déconnectée (1600) de l'au moins un feuillet (60) de la structure de feuillet (14) est située au niveau de la ligne de fixation près d'un périmètre extérieur (96).

13. Valvule prothétique selon l'une quelconque des revendications 6 à 12, dans laquelle la valvule et le cadre (12) sont configurés pour être radialement repliables jusqu'à un état replié ou serti pour l'introduction dans le corps sur un cathéter de placement et radialement déployés jusqu'à un état déployé pour l'implantation de la valvule à un endroit désiré dans le corps.

14. Valvule prothétique selon l'une quelconque des revendications 6 à 13, dans laquelle la structure de feuillet (14) est formée de tissu péricardique bovin ou de matériaux synthétiques biocompatibles.
